# EUROPÄISCHE PATENTSCHRIFT

(11) **EP 4 447 830 B1**
(45) Veröffentlichungstag und Bekanntmachung des Hinweises auf die Patenterteilung: **28.05.2025**
(21) Anmeldenummer: 22818268.9
(22) Anmeldetag: 15.11.2022
(51) Int. Cl.: A61B 17/80

(54) **FIXIERANORDNUNG**
FIXING ARRANGEMENT
AGENCEMENT DE FIXATION

(43) Veröffentlichungstag der Anmeldung: 23.10.2024
(73) Patentinhaber: Mühlenkreiskliniken Aör, 32429 Minden (DE)
(72) Erfinder: ZEICHEN, Johannes, 32429 Minden (DE)
(74) Vertreter: Schober, Mirko
(86) Internationale Anmeldenummer: PCT/EP2022/081929
(87) Internationale Veröffentlichungsnummer: WO 2024/104566

(56) Entgegenhaltungen:
- EP-A1- 2 340 777
- WO-A2-2012/119112
- DE-A1- 102019 110 633
- DE-A1- 102020 116 610
- US-A- 4 003 376

## Beschreibung

### TECHNISCHES GEBIET

Die Erfindung betrifft eine Fixieranordnung zur Fixierung eines Knochens oder eines knochenersetzenden oder knochenstabilisierenden Bauteils.

### STAND DER TECHNIK

Gerade bei älteren Patienten kommt es oftmals bereits schon bei geringen äußeren Belastungen zu Frakturen. Ein Grund dafür ist die Schwächung der Knochensubstanz durch Osteoporose. Entsprechende Frakturen müssen operativ fixiert werden, wobei in der Regel sogenannte Knochenplatten oder Stabilisierungsplatten eingesetzt werden, die mit dem Knochen verschraubt werden. So ist beispielsweise bekannt, einen Knochen mit zwei solchen winkelstabilen Stabilisierungsplatten zu fixieren, wie dies beispielsweise aus DE 10 2019 110 633 A1 bekannt ist. Zusätzlich zur Fixierung der einzelnen Knochenplatten mittels Schrauben, die in den Knochen hineingeschraubt werden, werden auch gegenüberliegende Knochenplatten mittels knochenpenetrierender Schrauben verbunden. Durch diese Maßnahme, die operativ sehr aufwendig ist, wird der Knochen allerdings enorm geschwächt, sodass eine entsprechende Fixiervorrichtung bei äußeren Krafteinwirkungen versagen kann.

Es ist zum Beispiel auch aus DE 10 2020 116 610 A1 bekannt, Knochenplatten mittels um den Knochen herum geführten Schlingen zu fixieren. Aus US 4,003,376 ist eine Anordnung bekannt, bei der eine einzige Stabilisierungsplatte über um diese herumgelegte, gebogene Gewindestangen gehalten wird. Als Widerlager dienen auf der anderen Seite des Knochens entsprechende Befestigungsabschnitte, an denen jeweils ein Ende der jeweiligen gebogenen Gewindestange mit einer Kontermutter fixiert ist. Diese Anordnung hat einen ähnlichen Effekt, wie die oben genannten Schlingen. Nachteilig hieran ist jedoch, dass entsprechende Schlingen, die zumeist aus Draht bestehen, während des operativen Eingriffs aufwendig durch die von Knochen umgebenden Weichteile geführt und anschließend zusammengezogen und fixiert werden müssen. Zumeist müssen mehrere solcher Schlingen eingesetzt werden. Dies kann die Blutversorgung des Knochens verringern, was letztlich das Verheilen der Fraktur behindert.

Aus EP 2 340 777 A1 ist eine Osteosyntheseplatte bekannt, die aus drei Teilabschnitten besteht. Zwei Enden sind mit Stabilisierungsplatten vergleichbar, die durch den Mittenabschnitt verbunden sind. Diese Platte ist einstückig ausgeführt. Diese Anordnung ist nachteilig, da sie für lediglich eine spezielle Knochengeometrie geeignet ist. Zudem gibt es bei dieser Anordnung operative Schwierigkeiten. Wird zum Beispiel ein Bruch am Oberschenkel mit dieser Anordnung fixiert, so müssen die beiden Stabilisierungsplatten an der äußeren und inneren Seite des Oberschenkels angebracht werden. Durch die einstimmige Ausbildung ist die relative Lage beider Stabilisierungsplatten vorgegeben, eine intraoperative Korrektur dieser Anordnung ist nicht mehr möglich.

### DIE ERFINDUNG

Aufgabe der vorliegenden Erfindung ist es daher, eine Fixieranordnung zur Verfügung zu stellen, mit der die oben geschilderten Nachteile nicht auftreten und insbesondere eine weitere Schwächung des Knochens vermieden wird.

Gelöst wird diese Aufgabe durch eine Fixieranordnung mit den Merkmalen des Anspruchs 1. Vorteilhafte Ausführungsformen finden sich in den abhängigen Ansprüchen.

Erfindungsgemäß wird eine Fixieranordnung zur Fixierung eines Knochens oder eines knochenersetzenden oder knochenstabilisierenden Bauteils vorgeschlagen, welche wenigstens zwei Stabilisierungsplatten und wenigstens ein Kraftübertragungselement umfasst, welches dazu ausgelegt ist, Kräfte von der einen Stabilisierungsplatte auf die wenigstens eine weitere Stabilisierungsplatte zu übertragen. Die Stabilisierungsplatten sind dabei über das wenigstens eine Kraftübertragungselement so miteinander verbunden oder verbindbar, dass das Kraftübertragungselement mit einem ersten Abschnitt an der einen Stabilisierungsplatte befestigbar oder befestigt ist und mit einem zweiten Abschnitt an der weiteren Stabilisierungsplatte befestigbar oder befestigt oder integral ausgebildet ist. Auf diese Weise kann das Kraftübertragungselement im mechanischen Sinne als sogenannte feste Einspannung angesehen werden. Dadurch ist das Kraftübertragungselement in der Lage, Kräfte, also Drehmomente und linear wirkende Kräfte, aufzunehmen und zu übertragen. Dies hat zur Folge, dass auf eine Stabilisierungsplatte wirkende Kräfte in das Kraftübertragungselement und mithin auf die weitere Stabilisierungsplatte übertragen werden. Durch die mechanische Kopplung der Stabilisierungsplatten über das erfindungsgemäße Kraftübertragungselement können äußere Kräfte, die auf den durch die Fixieranordnung fixierten Knochen einwirken, über die vorhandenen Stabilisierungsplatten verteilt werden. Durch diese Kräfteverteilung ist die erfindungsgemäße Fixieranordnung insgesamt schonender für den zu fixierenden Knochen oder das zu fixierende Bauteil.

Das mit den Stabilisierungsplatten verbundene Kraftübertragungselement kann dabei vollständig außerhalb eines mit der Fixieranordnung zu fixierenden Knochens oder eines mit der Fixieranordnung zu fixierenden knochenersetzenden oder knochenstabilisierenden Bauteils angeordnet sein. Auf diese Weise werden vom Kraftübertragungselement aufgenommene Kräfte nicht unmittelbar an den zu fixierenden Knochen abgegeben. Insbesondere kann dadurch Druck auf den zu fixierenden Knochen, der die Durchblutung im Knochen stören kann, vermieden werden.

Die Stabilisierungsplatten können also über das wenigstens eine Kraftübertragungselement so miteinander verbunden oder verbindbar sein, dass ein mit der Fixieranordnung fixierter Knochen oder ein mit der Fixieranordnung fixiertes knochenersetzendes oder knochenstabilisierendes Bauteil vom Kraftübertragungselement und den Stabilisierungsplatten teilweise manschettenartig umschlossen wird.

An dieser Stelle sei darauf hingewiesen, dass erfindungsgemäß auch eine Mehrzahl Kraftübertragungselemente eingesetzt werden kann, die alle so ausgebildet sein können, wie das nachfolgende Kraftübertragungselement. Auf diese Weise wird die Verteilung der einwirkenden Kräfte auf die Stabilisierungsplatten gleichmäßig von mehreren Kraftübertragungselementen übernommen.

Zusätzlich umfasst die erfindungsgemäße Fixieranordnung ein oder eine Mehrzahl Verbindungsmittel, mit denen die Stabilisierungsplatten und das wenigstens eine Kraftübertragungselement aneinander befestigbar oder befestigt sind und die zur kraftübertragenden Verbindung der Stabilisierungsplatten über das Kraftübertragungselement ausgebildet sind.

Es ist erfindungsgemäß vorgesehen, dass der obengenannte erste Abschnitt, bevorzugt ein erstes Ende, des Kraftübertragungselements und über ein erstes Verbindungsmittel an der einen Stabilisierungsplatte und der oben genannte zweite Abschnitt, bevorzugt ein zweites Ende, des Kraftübertragungselements und über ein vom ersten Verbindungsmittel verschiedenes und mit diesem nicht unmittelbar mechanisch gekoppeltes zweites Verbindungsmittel an der weiteren Stabilisierungsplatte befestigbar oder befestigt ist. Nicht unmittelbar mechanisch gekoppelt bedeutet in diesem Zusammenhang, dass beide Verbindungsmittel verschiedene Bauteile sind und diese miteinander nicht unmittelbar verbunden sind. Die Verbindungsmittel sind vorteilhafterweise, bevorzugt winkelstabile, Schrauben.

Bevorzugt ist das Kraftübertragungselement so ausgelegt, dass es Zug- und Druckkräfte aufnehmen kann. Darüber hinaus ist es von Vorteil, wenn das Kraftübertragungselement so ausgelegt und mit den Stabilisierungsplatten verbindbar oder verbunden ist, dass es um wenigstens eine Achse wirkende Drehmomente und/oder lineare Krafteinwirkungen in wenigstens zwei Raumrichtungen überträgt.

Je weniger das Kraftübertragungselement also selbst bei Einwirkung von linearen Kräften oder Drehmomenten verformt wird, desto besser kann es auftretende Kräfte auf die mit ihm verbundenen Stabilisierungsplatten verteilen.

Die Beschaffenheit des Kraftübertragungselements sollte entsprechend den zu erwartenden mechanischen Belastungen gewählt werden. Was die Materialwahl betrifft, so ist das wenigstens eine Kraftübertragungselement vorteilhafterweise aus einem Metallmaterial, bevorzugt Stahl oder Titan, ausgebildet.

Hinsichtlich der Geometrie ist diese so abzustimmen, dass die zu erwartenden Kräfte oder Drehmomente aufgenommen und abgeleitet werden können. Vorteilhafte Ausführungsformen sehen vor, dass das wenigstens eine Kraftübertragungselement eine Streifen-, Bügel- oder Klammerform aufweist. Diese Formen bringen schon eine gewisse Eigensteifigkeit mit, wenn jedenfalls die Materialstärke entsprechend gewählt wird, so dass solche Bauteile Kräfte zumindest in den hier erforderlichen Raumrichtungen/Drehrichtungen aufnehmen und ableiten können.

Die oben genannten Abschnitte des Kraftübertragungselements können zur, bevorzugt formschlüssigen, Anlage an die Stabilisierungsplatten ausgelegt sein. Auf diese Weise lassen sich Kräfte von einer Stabilisierungsplatte noch effektiver auf eine weitere Stabilisierungsplatte übertragen. Bevorzugt ist auch vorgesehen, dass die Abschnitte des Kraftübertragungselements zur Anbringung oder Durchführung eines Verbindungsmittels ausgebildet sind.

Bevorzugt umfassen die Stabilisierungsplatten jeweils wenigstens eine Aufnahme, bevorzugt ein Gewindeloch, zur Aufnahme der Verbindungsmittel. Dabei kann es hilfreich sein, dass das Kraftübertragungselement, bevorzugt ein Innengewinde aufweisende, Öffnungen zur Durchführung von Verbindungsmitteln aufweist, die mit den Aufnahmen der Stabilisierungsplatten fluchten. In einem bevorzugten Fall werden Verbindungsmittel in Gestalt von Schrauben durch die Öffnungen hindurchgeführt und in die jeweilige Aufnahme in der Stabilisierungsplatte eingeschraubt.

Die erfindungsgemäße Fixiervorrichtung wird nach folgendem Verfahren (das Verfahren bildet keinen Teil der beanspruchten Erfindung) installiert:
a. ein zu fixierender Knochen oder ein knochenersetzendes oder knochenstabilisierendes Bauteil wird in einer Solllage fixiert,
b. die Stabilisierungsplatten werden an den vorgesehenen Stellen, bevorzugt mittels Schrauben, am Knochen oder dem knochenersetzenden oder knochenstabilisierenden Bauteil befestigt,
c. vor oder nach Schritt b. wird wenigstens ein oben beschriebenes Kraftübertragungselement mittels Verbindungsmitteln an den Stabilisierungsplatten befestigt.

### KURZBESCHREIBUNG DER ZEICHNUNGSABBILDUNGEN

Die Erfindung wird nachfolgend anhand der Figuren 1-5 näher erläutert.
- Figur 1 -: zeigt eine Seitenansicht eines mit der erfindungsgemäßen Fixieranordnung fixierten Knochens,
- Figur 2 -: zeigt eine Schnittansicht entlang der Linie A-A aus Figur 1 mit einer ersten Befestigungsmöglichkeit des erfindungsgemäßen Kraftübertragungselements,
- Figur 3 -: zeigt eine Schnittansicht entlang der Linie A-A aus Figur 1 mit einer zweiten Befestigungsmöglichkeit des erfindungsgemäßen Kraftübertragungselements,
- Figur 4 -: zeigt eine perspektivische Ansicht einer möglichen Ausführungsform des erfindungsgemäßen Kraftübertragungselements,
- Figur 5 -: zeigt eine perspektivische Ansicht einer weiteren Ausführungsform des erfindungsgemäßen Kraftübertragungselements.

### BESTER WEG ZUR AUSFÜHRUNG DER ERFINDUNG

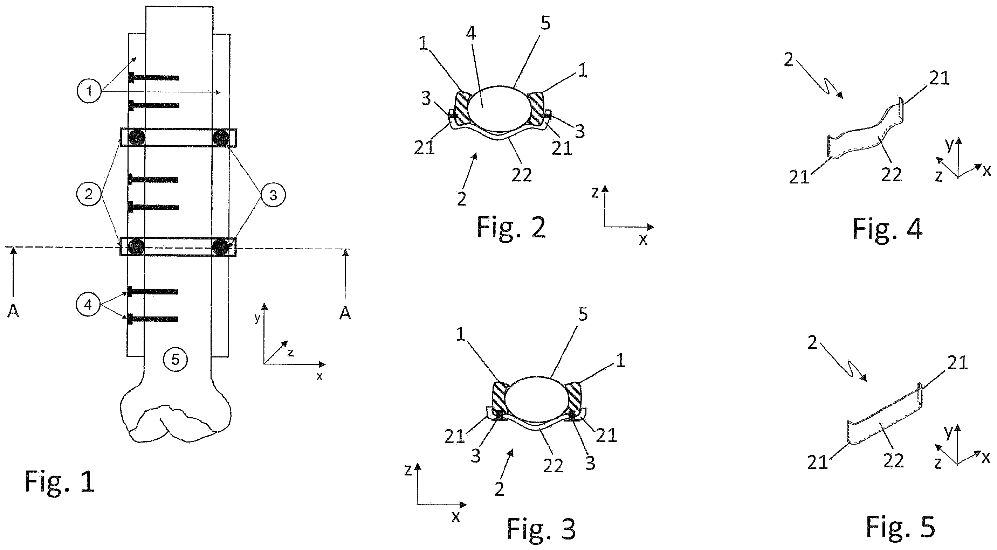

Figur 1 zeigt ein Anwendungsbeispiel für eine erfindungsgemäße Fixieranordnung. Der zu versorgende Knochen 5 wird rechts und links von zwei Stabilisierungsplatten 1 eingefasst. Dabei ist eine der Stabilisierungsplatten 1 (in der Fig. die linke) an dem Knochen mittels Fixierelementen 4, die bevorzugt Knochenschrauben sind, fixiert. Genauer ausgedrückt ist der Knochen 5 bzw. die zu versorgenden Knochenfragmente an der linken Stabilisierungsplatte mittels Fixierelementen 4 fest angeordnet. Unterstützt wird eine solche Fixierung durch eine weitere Stabilisierungsplatte 1, die der ersten Stabilisierungsplatte 1 gegenüber an dem Knochen 5 angeordnet wird, um diesen von der anderen Seite her zu stabilisieren. Grundsätzlich könnten auch beide Stabilisierungsplatten 1 mittels Knochenschrauben am Knochen fixiert sein.

Dabei wird die zweite Stabilisierungsplatte 1 mittels Kraft-übertragungselementen 2 mit der ersten Stabilisierungsplatte 1 verbunden. Dies erfolgt mit Verbindungsmitteln 3, bei denen es sich bevorzugt um Schrauben handelt, mit denen die Kraftübertragungselemente 2 fest mit der jeweiligen Stabilisierungsplatte 1 verschraubt werden. Mit den Kraftübertragungselementen 2 werden Kräfte von einer zur anderen Stabilisierungsplatte 1 übertragen, ohne dass diese durch den Knochen 5 geleitet werden müssen. Eine direkte Schraubverbindung durch den Knochen würde zwar diese direkte Kraftübertragung ebenfalls erfüllen, würde dabei allerdings den Knochen 5, durch den die Schraube geführt werden müsste, weiter schwächen.

Figur 2 zeigt einen Schnitt durch die Schnittlinie A-A der Figur 1. Es zeigt den Knochen 5, der links und rechts von den Stabilisierungsplatten 1 eingefasst ist. Die rechte Stabilisierungsplatte 1 ist über ein Kraftübertragungselement 2 mit der linken Stabilisierungsplatte 1 verbunden. Die beiden abgewinkelten Endabschnitte 21 des Kraftübertragungselements 2 sind mit einem mittleren Abschnitt 22, der in dieser Ausführungsform mit einer Auswölbung versehen ist, verbunden. Die Abwinkelung der Endabschnitte 21 ist so ausgeführt, dass die Endabschnitte 21 des Kraftübertragungselements 2 formschlüssig oder zumindest nahezu formschlüssig an den Seiten der Stabilisierungsplatte 1 anliegen. In der gezeigten Ausführungsform ist in dem abgewinkelten Abschnitt des Endabschnitts 21 eine Öffnung, bevorzugt eine Gewindeöffnung, angeordnet, die mit einem entsprechenden Gewindeloch in der Stabilisierungsplatte 1 zum Fluchten gebracht wird und dann mit einem Verbindungselement 3, bevorzugt einer Schraube, an der Stabilisierungsplatte 1 fixiert wird. Durch eine solche Befestigung werden auf eine Stabilisierungsplatte 1 einwirkende Kräfte, wie beispielsweise Druck-, Zug- oder Torsionskräfte, auf die andere Stabilisierungsplatte 1 übertragen, ohne den Knochen 5 bzw. die Knochenfraktur zu belasten.

Figur 3 zeigt eine zweite Befestigungsmöglichkeit des Kraftübertragungselements 2 an der Stabilisierungsplatte 1. Die Abwinkelung der Endabschnitte 21 des Kraftübertragungselements 2 ist kürzer ausgeführt. Das Gewindeloch befindet sich in dieser Ausführungsform, vom Mittelabschnitt 22 aus betrachtet, vor der Abwinkelung, so dass das Kraftübertragungselement 2 an der Schmalseite der Stabilisierungsplatte 1 mit einen Verbindungsmittel 3 fixiert wird.

Es sind auch andere Befestigungsmöglichkeiten des Kraftübertagungselements an der Stabilisierungsplatte 1 möglich. Beispielsweise ist es möglich, dass das Kraftübertragungselement 2 stoffschlüssig mit einer der Stabilisierungsplatten 1 verbunden ist, während es mit der anderen Stabilisierungsplatte 1 lösbar formschlüssig verbunden wird; auch ist es möglich, dass das Kraftübertragungselement einstückig mit einer der Stabilisierungsplatten 1 gebildet wird.

Figur 4 zeigt eine Ausführungsform eines erfindungsgemäßen Kraftübertragungselements 2. Der, ggf. leicht nach außen gewölbte, Mittelabschnitt 22 verbindet die beiden abgewinkelten Endabschnitte 21, mit denen das Kraftübertragungselement 2 an den Stabilisierungsplatten 1 (nicht gezeigt) fixiert wird. Es ist auch keine Öffnung für das Verbindungsmittel 3 gezeigt. Letztendlich müssen die Kraftübertragungen zwischen den Stabilisierungsplatten 1 immer über die Kraftübertragungselemente 2 erfolgen und möglichst nicht über den Knochen 5, welcher ja gerade durch eine solche Fixieranordnung entlastet werden soll. Die Steifigkeit des Kraftübertragungselements 2 kann noch zusätzlich dadurch erhöht werden, dass die Längsseiten des Kraftübertragungselements 2 verstärkt sind oder das Kraftübertragungselement 2 hinsichtlich seiner Materialstärke hinreichend dick ausgebildet ist.

Figur 5 zeigt eine weitere denkbare Ausführungsform eines Kraftübertragungselements 2. Während die Endabschnitte 21, die an den Stabilisierungsplatten 1 angeordnet werden, gleich oder nahezu gleich ausgeführt sind, wird der Mittelabschnitt 22 von einem geraden Abschnitt gebildet. Dieser zeigt weniger Federneigung gegenüber der Ausführungsform von Figur 4 bzgl. der Druck- und Zugkraft. Auch hier kann eine Torsionsstabilisierung durch das Bördeln des Randes, Verstärkung des Materials oder andere geeignete Maßnahmen erreicht werden.

## Patentansprüche

1. Fixieranordnung zur Fixierung eines Knochens (5) oder eines knochenersetzenden oder knochenstabilisierenden Bauteils, wobei die Fixieranordnung wenigstens zwei Stabilisierungsplatten (1) und wenigstens ein Kraftübertragungselement (2) umfasst, welches dazu ausgelegt ist, Kräfte von der einen Stabilisierungsplatte (1) auf die wenigstens eine weitere Stabilisierungsplatte (1) zu übertragen, wobei die Stabilisierungsplatten (1) über das wenigstens eine Kraftübertragungselement (2) dergestalt miteinander verbindbar oder verbunden sind, dass das Kraftübertragungselement (2) mit einem ersten Abschnitt (21) an der einen Stabilisierungsplatte (1) befestigbar oder befestigt ist und mit einem zweiten Abschnitt (21) an der weiteren Stabilisierungsplatte (1) befestigbar oder befestigt oder integral ausgebildet ist,
**dadurch gekennzeichnet,**
**dass** die Fixieranordnung Verbindungsmittel (3) umfasst, mit denen die Stabilisierungsplatten (1) und das wenigstens eine Kraftübertragungselement (2) aneinander befestigbar oder befestigt sind und die zur kraftübertragenden Verbindung der Stabilisierungsplatten (1) über das Kraftübertragungselement (2) ausgebildet sind, wobei der erste Abschnitt (21), bevorzugt ein erstes Ende, des Kraftübertragungselements (2) über ein erstes Verbindungsmittel (3) an der einen Stabilisierungsplatte (1) und der zweite Abschnitt (22), bevorzugt ein zweites Ende, des Kraftübertragungselements (2) über ein vom ersten Verbindungsmittel verschiedenes und mit diesem nicht mechanisch gekoppeltes zweites Verbindungsmittel (3) an der weiteren Stabilisierungsplatte (1) befestigbar oder befestigt ist.

2. Fixieranordnung nach Anspruch 1,
**dadurch gekennzeichnet,**
**dass** das mit den Stabilisierungsplatten verbundene Kraftübertragungselement (2) vollständig außerhalb eines mit der Fixieranordnung zu fixierenden Knochens (5) oder eines mit der Fixieranordnung zu fixierenden knochenersetzenden oder knochenstabilisierenden Bauteils anordenbar ist.

3. Fixieranordnung nach einem der vorigen Ansprüche,
**dadurch gekennzeichnet,**
**dass** das Kraftübertragungselement (2) so ausgelegt ist, dass es Zug- und Druckkräfte aufnehmen kann.

4. Fixieranordnung nach einem der vorigen Ansprüche,
**dadurch gekennzeichnet,**
**dass** das Kraftübertragungselement (2) so ausgelegt und mit den Stabilisierungsplatten (1) verbindbar oder verbunden ist, dass es um wenigstens eine Achse wirkende Drehmomente überträgt.

5. Fixieranordnung nach einem der vorigen Ansprüche,
**dadurch gekennzeichnet,**
**dass** das Kraftübertragungselement (2) so ausgelegt und mit den Stabilisierungsplatten (1) verbindbar oder verbunden ist, dass es lineare Krafteinwirkungen in wenigstens zwei Raumrichtungen überträgt.

6. Fixieranordnung nach einem der vorigen Ansprüche,
**dadurch gekennzeichnet,**
**dass** die Verbindungsmittel (3), bevorzugt winkelstabile, Schrauben sind.

7. Fixieranordnung nach einem der vorigen Ansprüche,
**dadurch gekennzeichnet,**
**dass** das wenigstens eine Kraftübertragungselement (2) aus einem Metallmaterial, bevorzugt Stahl oder Titan, ausgebildet ist.

8. Fixieranordnung nach einem der vorigen Ansprüche,
**dadurch gekennzeichnet,**
**dass** das wenigstens eine Kraftübertragungselement (2) eine Streifen-, Bügel- oder Klammerform aufweist.

9. Fixieranordnung nach einem der vorigen Ansprüche,
**dadurch gekennzeichnet,**
**dass** die Abschnitte (21, 22) des Kraftübertragungs-elements (2) zur, bevorzugt formschlüssigen, Anlage an die Stabilisierungsplatten ausgelegt sind.

10. Fixieranordnung nach einem der vorigen Ansprüche,
**dadurch gekennzeichnet,**
**dass** die Abschnitte (21, 22) des Kraftübertragungselements (2) zur Anbringung oder Durchführung eines Verbindungsmittels (3) ausgebildet sind.

11. Fixieranordnung nach Anspruch 9 oder 10,
**dadurch gekennzeichnet,**
**dass** die Stabilisierungsplatten (1) jeweils wenigstens eine Aufnahme, bevorzugt ein Gewindeloch, zur Aufnahme der Verbindungsmittel (3) umfassen.

12. Fixieranordnung nach Anspruch 11,
**dadurch gekennzeichnet,**
**dass** das Kraftübertragungselement (2), bevorzugt ein Innengewinde aufweisende, Öffnungen zur Durchführung von Verbindungsmitteln (3) aufweist, die mit den Aufnahmen der Stabilisierungsplatten (1) fluchten.

## Claims

1. Fixing arrangement for fixing a bone (5) or a bone replacement or bone-stabilizing part wherein the fixing arrangement comprises at least two stabilizing plates (1) and at least one force transfer element (2) which is configured to transfer forces from one stabilizing plate (1) to the at least one further stabilizing plate (1), wherein the stabilizing plates (1) are or can be connected to one another via the at least one force transfer element (2) in such a way that the force transfer element (2) is or can be fixed with a first section (21) on the one stabilizing plate (1) and is or can be fixed, or is formed integral, with a second section (21) on the further stabilizing plate (1)
**characterised in that**
the fixing arrangement comprises connecting means (3) with which the stabilizing plates (1) and the at least one force transfer element (2) are or can be fastened to one another and which are designed for the force-transferring connection of the stabilizing plates (1) by way of the force transfer element (2), wherein the first section (21), preferably a first end, of the force transfer element (2) is or can be fastened via a first connecting means (3) on the one stabilizing plate (1), and the second section (22), preferably a second end, of the force transfer element (2) is or can be fastened on the further stabilizing plate (1) via a second connecting means (3) which is different from the first connecting means and is non-mechanically coupled to the latter.

2. Fixing arrangement according to Claim 1
**characterised in that**
the force transfer element (2) connected to the stabilizing plates can be arranged entirely outside of a bone (5), that is to be fixed with the fixing arrangement, or entirely outside of a bone replacement or bone-stabilizing part that is to be fixed with the fixing arrangement.

3. Fixing arrangement according to one of the preceding claims
**characterised in that**
the force transfer element (2) is designed so that it can take up tensile and compressive forces.

4. Fixing arrangement according to one of the preceding claims
**characterised in that**
the force transfer element (2) is designed and is or can be connected with the stabilizing plates (1) so that it transfers torques acting about at least one axis.

5. Fixing arrangement according to one of the preceding claims
**characterised in that**
the force transfer element (2) is designed and is or can be connected to the stabilizing plates (1) so that it transfers linear forces acting in at least two spatial directions.

6. Fixing arrangement according to one of the preceding claims
**characterised in that**
the connecting means (3) are screws, preferably fixed-angle screws.

7. Fixing arrangement according to one of the preceding claims
**characterised in that**
the at least one force transfer element (2) is made from a metal material, preferably from steel or titanium.

8. Fixing arrangement according to one of the preceding claims
**characterised in that**
the at least one force transfer element (2) has a strip-shape, U-shape, or bracket-shape.

9. Fixing arrangement according to one of the preceding claims
**characterised in that**
the sections (21, 22) of the force transfer element (2) are designed for preferably form-locking abutment against the stabilizing plates.

10. Fixing arrangement according to one of the preceding claims
**characterised in that**
the sections (21, 22) of the force transfer element (2) are designed for attaching or guiding a connecting means (3).

11. Fixing arrangement according to Claim 9 or 10
**characterised in that**
the stabilizing plates (1) each comprise at least one socket, preferably a threaded hole, for receiving the connecting means (3).

12. Fixing arrangement according to Claim 11
**characterised in that**
the force transfer element (2) has openings, preferably having an internal thread, for guiding the connecting means (3) which align flush with the sockets of the stabilizing plates (1).

## Revendications

1. Agencement de fixation pour fixer un os (5) ou un composant de remplacement d'os ou de stabilisation osseuse, dans lequel l'agencement de fixation comprend au moins deux plaques de stabilisation (1) et au moins un élément de transmission de force (2), qui est conçu pour transmettre des forces d'une plaque de stabilisation (1) à l'au moins un autre plaque de stabilisation (1), dans lequel les plaques de stabilisation (1) sont reliées ou peuvent être reliées l'une à l'autre par l'intermédiaire de l'au moins un élément de transmission de force (2) de telle sorte que l'élément de transmission de force (2) est fixé ou peut être fixé avec une première section (21) à l'une des plaques de stabilisation (1) et avec une seconde section (22) à l'autre plaque de stabilisation (1) ou est formé d'un seul tenant avec ces dernières,
**caractérisé en ce**
**que** l'agencement de fixation comporte des moyens de liaison (3) par l'intermédiaire desquels les plaques de stabilisation (1) et l'au moins un élément de transmission de force (2) sont fixés ou peut être fixés entre eux, et qui sont conçus par l'élément de transmission de force (2) pour une liaison de transmission de force des plaques de stabilisation (1), dans lequel la première section (21), de préférence une première extrémité, de l'élément de transmission de force (2) est fixée ou peut être fixée à l'un des plaques de stabilisation (1) par un premier moyen de liaison (3), et la seconde section (22), de préférence une deuxième extrémité, de l'élément de transmission de force (2), est fixée ou peut être fixée à l'autre plaque de stabilisation (1) par un second moyen de liaison (3) différent du premier moyen de liaison et non couplé mécaniquement avec celui-ci.

2. Agencement de fixation selon la revendication 1,
**caractérisé en ce**
**que** l'élément de transmission de force (2) lié aux plaques de stabilisation (1) peut être agencé complètement à l'extérieur d'un os (5) à fixer avec l'agencement de fixation ou d'un composant de remplacement d'os ou de stabilisation osseuse à fixer avec l'agencement de fixation.

3. Agencement de fixation selon l'une quelconque des revendications précédentes,
**caractérisé en ce**
**que** l'élément de transmission de force (2) est conçu pour absorber des forces de traction et de compression.

4. Agencement de fixation selon l'une quelconque des revendications précédentes,
**caractérisé en ce**
**que** l'élément de transmission de force (2) est conçu et peut être ou est lié aux plaques de stabilisation (1) pour transmettre des couples de rotation agissant autour d'au moins un axe.

5. Agencement de fixation selon l'une quelconque des revendications précédentes,
**caractérisé en ce**
**que** l'élément de transmission de force (2) est conçu et peut être ou est lié aux plaques de stabilisation (1) pour transmettre des forces linéaires dans au moins deux directions spatiales.

6. Agencement de fixation selon l'une quelconque des revendications précédentes,
**caractérisé en ce**
**que** les moyens de liaison (3) sont des vis, de préférence des vis avec une stabilité angulaire.

7. Agencement de fixation selon l'une quelconque des revendications précédentes,
**caractérisé en ce**
**que** l'au moins un l'élément de transmission de force (2) est formé d'un matériau métallique, de préférence de l'acier ou de la titane.

8. Agencement de fixation selon l'une quelconque des revendications précédentes,
**caractérisé en ce**
**que** l'au moins un l'élément de transmission de force (2) comporte une forme de la bande, de l'étrier ou de l'agrafe.

9. Agencement de fixation selon l'une quelconque des revendications précédentes,
**caractérisé en ce**
**que** les sections (21, 22) de l'élément de transmission de force (2) sont conçues pour un appui à engagement positif aux plaques de stabilisation (1).

10. Agencement de fixation selon l'une quelconque des revendications précédentes,
**caractérisé en ce**
**que** les sections (21, 22) de l'élément de transmission de force (2) sont conçues pour la fixation ou le passage d'un moyen de liaison (3).

11. Agencement de fixation selon la revendication 9 ou 10,
**caractérisé en ce**
**que** les plaques de stabilisation (1) comporte chacune au moins une réception, de préférence un trou fileté, pour recevoir les moyens de liaison (3).

12. Agencement de fixation selon la revendication 11,
**caractérisé en ce**
**que** l'élément de transmission de force (2) comporte des orifices, de préférence comprenant un filetage intérieur, pour le passage des moyens de liaison (3) qui sont alignés avec des réceptions des plaques de stabilisation (1).
